# EUROPEAN PATENT SPECIFICATION

(11) **EP 2 306 821 B1**
(45) Date of publication and mention of the grant of the patent: **01.10.2014**
(21) Application number: 09767399.0
(22) Date of filing: 29.05.2009
(51) Int. Cl.: A01N 37/44, A01P 1/00, A01N 25/34

(54) **USE OF ZINC CHELATORS COMPRISING DTPA TO INHIBIT BIOFILM FORMATION**
VERWENDUNG VON ZINK-CHELIERATOREN ENTHALTEND DTPA ZUM HEMMEN DER BIOFILMBILDUNG
INHIBITION DE LA FORMATION DE BIOFILMS À L'AIDE DE CHÉLATEURS DE ZINC COMPRENANT LE DTPA

(30) Priority: 30.05.2008 US 57267
(43) Date of publication of application: 13.04.2011
(73) Proprietor: University Of Cincinnati, Cincinnati, OH 45221-0829 (US)
(72) Inventor: HERR, Andrew, B., Cincinnati OH 45248 (US); CONRADY, Deborah, Gail, Cincinnati OH 45245 (US); BRESCIA, Cristin, C., Canton OH 44718 (US); WARD, Stefanie, L., Cincinnati OH 45233 (US)
(74) Representative: Kehoe, Laura Ellen
(86) International application number: PCT/US2009/045623
(87) International publication number: WO 2009/155088

(56) References cited:
- WO-A1-94/10838
- WO-A2-2007/068938
- US-A- 5 688 516
- US-A1- 2002 091 074
- US-A1- 2004 071 769
- R. M. Q. SHANKS: "Catheter lock solutions influence staphylococcal biofilm formation on abiotic surfaces", NEPHROLOGY DIALYSIS TRANSPLANTATION, vol. 21, no. 8, 1 August 2006 (2006-08-01) , pages 2247-2255, XP55019128, ISSN: 0931-0509, DOI: 10.1093/ndt/gfl170
- DUNNE ET AL: "The effects of magnesium, calcium, EDTA, and pH on the in vitro adhesion of Staphylococcus epidermidis to plastic.", MICROBIOLOGY AND IMMUNOLOGY, vol. 36, no. 10, 1 January 1992 (1992-01-01), pages 1019-1027, XP55019153, ISSN: 0385-5600
- STEVEN L. PERCIVAL ET AL: "Tetrasodium EDTA as a Novel Central Venous Catheter Lock Solution Against Biofilm", INFECTION CONTROL AND HOSPITAL EPIDEMIOLOGY, vol. 26, no. 6, 1 June 2005 (2005-06-01), pages 515-519, XP55019175, ISSN: 0899-823X, DOI: 10.1086/502577
- PAPAGEORGIOU A C ET AL: "Crystal structure of the superantigen enterotoxin C2 from Staphylococcus aureus reveals a zinc-binding site", STRUCTURE, CURRENT BIOLOGY LTD., PHILADELPHIA, PA, US, vol. 3, no. 8, 1 August 1995 (1995-08-01), pages 769-779, XP004587900, ISSN: 0969-2126, DOI: 10.1016/S0969-2126(01)00212-X
- PACITTI D F ET AL: "Characterization and overexpression of the gene encoding Staphylococcus aureus DNA polymerase III", GENE, ELSEVIER, AMSTERDAM, NL, vol. 165, no. 1, 7 November 1995 (1995-11-07), pages 51-56, XP004043181, ISSN: 0378-1119, DOI: 10.1016/0378-1119(95)00377-I
- DAJCS ET AL.: 'Lysostaphin Treatment of Methicillin-Resistant Staphylococcus aureus Keratitis in the Rabbit.' INVEST. OPHTHALMOL. VIS. SCI. vol. 41, no. 6, May 2000, pages 1432 - 1437, XP008141825
- HART J R ED - GERHARTZ W ET AL: "ETHYLENEDIAMINETETRAACETIC ACID AND RELATED CHELATING AGENTS", 1 January 1987 (1987-01-01), ULLMANN'S ENCYCLOPEDIA OF INDUSTRIAL CHEMISTRY. ETHANOLAMINES TO FIBERS, 4. SYNTHETIC ORGANIC; [ULLMANN'S ENCYCLOPEDIA OF INDUSTRIAL CHEMISTRY], WEINHEIM, VCH VERLAG, DE, PAGE(S) 95 - 97, XP002162686,
- STEPHEN G. WALKER ET AL: 'The immunoglobulin A1 proteinase from Streptococcus pneumoniae is inhibited by tetracycline compounds' FEMS IMMUNOLOGY & MEDICAL MICROBIOLOGY vol. 48, no. 2, 01 November 2006, pages 218 - 222, XP055067062 DOI: 10.1111/j.1574-695X.2006.00148.x ISSN: 0928-8244
- 'THE METALLOBIOCHEMISTRY OF ZINC ENZYMES', vol. 56, 01 January 1984, JOHN WILEY & SONS, INC. deel BERT L VALLEE ET AL: 'THE METALLOBIOCHEMISTRY OF ZINC ENZYMES', pages 283 - 430, XP055067090

## Description

The present invention relates to the fields of microbiology and biochemistry, and more specifically to methods for inhibiting the formation of a bacterial biofilm, wherein the targeted bacteria comprise at least one zinc adhesion module and wherein the bacteria are selected from the group consisting of Staphylococcus epidermis and Staphylococcus aureus and combinations thereof, the method comprising contacting the bacteria with an effective amount of at least one zinc chelator, whereby biofilm formation is inhibited characterized in that the at least one zinc chelator comprises DTPA.

It has recently been estimated that hospital-acquired (nosocomial) infections are the fourth-leading cause of death in the United States, affecting 2 million patients per year and causing over 100,000 annual deaths, with a total annual cost of over $30 billion. Staphylococcal species such as *S. epidermidis* and *S. aureus* are responsible for the majority of nosocomial infections; treatment of these infections is often made much more challenging by the tendency of staphylococci to form biofilms.

Biofilms are bacterial communities that adhere to biological or abiotic substrata, differentiate into micro- and macrocolonies, and produce an extracellular matrix typically comprised of polysaccharides and proteins. Bacteria in biofilms are resistant to antibiotics and host immune responses and are extremely difficult to eradicate. For example, device-related infections due to staphylococcal biofilms often require surgical removal of the implanted device, debridement of the surrounding tissue, and prolonged antibiotic treatment.

Staphylococcal infections are a substantial re-emerging threat to human health. Starting in the 1980's a dramatic increase in hospital-acquired infections has been observed, primarily associated with *S. epidermidis* and other coagulase-negative staphylococci. *S. epidermidis* alone is responsible for up to two-thirds of all central nervous system shunt- or catheter-related infections, as well as up to half of all infections of prosthetic heart valves or artificial joints. The nosocomial infections caused by staphylococci are often persistent and recurring, particularly in the case of infections of indwelling medical devices. Furthermore, device-related *S. epidermidis* biofilms can lead to endocarditis or septicemia. Likewise, *S. aureus* biofilm-related infections can become disseminated to other regions, which is a concern due to the large number of toxins and tissue-degrading enzymes released by *S. aureus.*

Industrially, biofilms can accumulate on a wide variety of substrates and interfere in a number of industrial and commercial applications, including pipeline systems, cooling water systems in power plants, refineries, chemical plants, air conditioning systems, and the like. If uncontrolled, biofouling caused by biofilm formation can interfere with operations, lower efficiency of systems, waste energy and resources, and adversely affect product quality.

A need exists to control and inhibit biofilm formation in medical and industrial applications. One area of current interest involves the use of metal chelation as a means of inhibiting biofilms. The present invention relates specifically to the chelation of zinc metal ions in biofilms comprised of bacteria having a zinc adhesion module.

Methods of inhibiting biofilm formation in medical and industrial settings have previously been developed using metal chelators, specifically iron chelators. For example, U.S. Pat. No. 6,267,979, issued Jul. 31, 2001, to Raad et al., discloses the use of metal chelators in combination with antifungal or antibiotic compositions for the prevention of biofouling in water treatment, pulp and paper manufacturing and oil field water flooding. U.S. Patent No. 7,314,857, issued Jan 1, 2008, to Madhyastha, discloses synergistic antimicrobial compositions for inhibiting biofilm formation using combinations of an iron-sequestering glycoprotein, a cationic peptide, and an iron chelating agent. U.S. Pat. No. 7,446,089, issued Nov. 4, 2008, to Singh et al., is also directed to methods of inhibiting biofilm formation by limiting the amount of iron available to a population of bacteria, such that biofilm formation can be inhibited. These disclosures generally target iron, a higher affinity metal ion. Indeed, the specified ranges disclosed in Madhyastha for metal chelation, such as 12.5 to 200 mg/L, would not be effective for zinc chelation in gram positive bacteria, as zinc is a lower affinity metal ion requiring relatively higher concentrations of chelating agents.

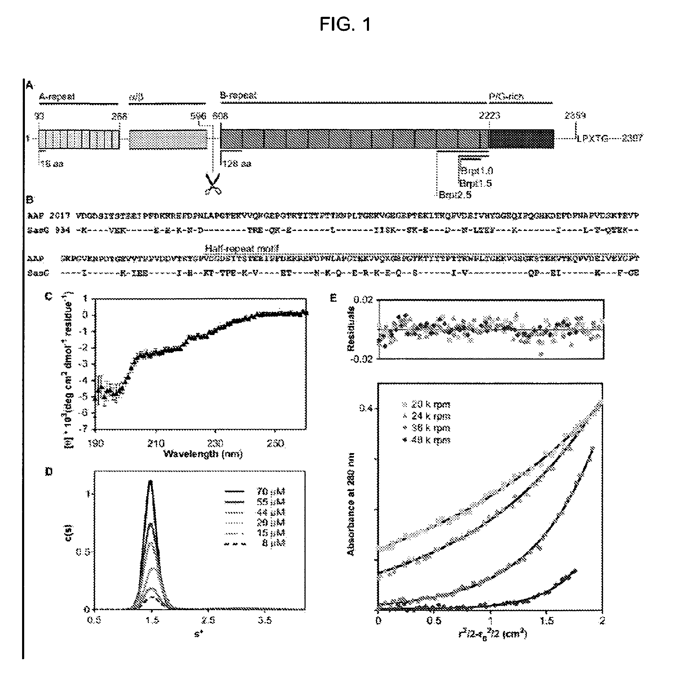

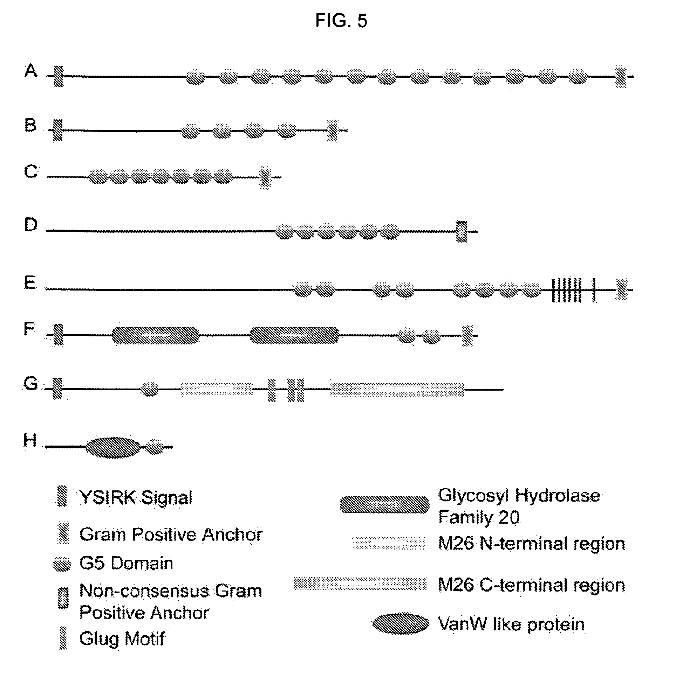

Staphylococcal biofilms are typically enmeshed within an extracellular polysaccharide matrix synthesized by proteins encoded by the *ica* operon; however, *ica-*negative staphylococcal biofilms have recently been described that rely on protein-protein interactions. The *S. epidermidis* surface protein Aap (Accumulation-associated protein) has been implicated in both polysaccharide-based and protein-based *S. epidermidis* biofilms. Aap contains an N-terminal A-repeat region with 11 degenerate 16 amino acid repeats, a putative globular domain ("α/β"), and a B-repeat region with a variable number (5 to 17) of nearly identical 128 amino acid repeats terminating in a conserved "half repeat" motif (Fig. 1 A). Rohde, H. et al., Polysaccharide intercellular adhesin or protein factors in biofilm accumulation of Staphylococcus epidermidis and Staphylococcus aureus isolated from prosthetic hip and knee joint infections, Biomaterials 28:1711-20 (2007). The repeated sequence element within the B-repeat region has recently been defined as a G5 domain, found in gram-positive surface proteins, Zn²⁺ metalloproteases, and other bacterial virulence factors (Fig. 5). The B-repeat region in Aap is followed by a collagen-like repeat and an LPXTG cell wall anchor sequence. A similar domain arrangement exists in the *S. aureus* homolog SasG (Fig. 1 B). Corrigan, R.M. et al., The role of Staphylococcus aureus surface protein SasG in adherence and biofilm formation, Microbiology 153:2435-46 (2007).

Proteolytic processing of Aap or SasG between the α/β and B-repeat regions induces the formation of protein-based biofilms in *S. epidermidis* and *S. aureus*; both proteinaceous and polysaccharide-based *S. epidermidis* biofilms are inhibited by anti-Aap antisera. Furthermore, addition of a large soluble Aap fragment containing the G5 and collagen-like regions inhibited formation of protein-based *S. epidermidis* biofilms. Rohde, H. et al., Induction of Staphylococcus epidermidis biofilm formation via proteolytic processing of the accumulation-associated protein by staphylococcal and host proteases, Mol. Microbiol. 55:1883-95 (2005). In *S. aureus*, protein-based biofilm formation was dependent on the number of G5 domains in SasG; five or more G5 domains were required to support biofilm formation. These studies suggested that the C-terminal halves of Aap and SasG containing the G5 domains are involved in bacterial interactions within staphylococcal biofilms, but the precise molecular mechanism was unclear.

Given the serious medical, industrial, and environmental problems associated with bacterial biofilms, the need persists to develop targeted approaches to inhibit biofilm formation.

US 5,688,516 A describes compositions and methods of employing compositions in flushing and coating medical devices. The compositions include selected combinations of a chelating agent, an anti-coagulant, or antithrombotic agent, with a non-glycopeptide antimicrobial agent, such as the tetracycline antibiotics. Methods of using these compositions for coating a medical device and for inhibiting catheter infection are described.

The present application discloses a biophysical characterization of a G5 domain-containing B-repeat region from Aap, revealing that it is a zinc (Zn²⁺)-dependent adhesion module ("zinc adhesion module") responsible for intercellular interaction in staphylococcal biofilms. This zinc adhesion module has been identified in a variety of bacteria, including gram positive bacteria, and provides a specific target for zinc chelation and biofilm inhibition in biofilms comprised of bacteria having a G5 domain.

Surprisingly, it has been found that zinc chelation inhibits formation of both *S. epidermidis* and methicillin-resistant *S. aureus* biofilms and supplementation with additional zinc in the physiological range reverses the effect. This reversible effect identified in bacteria having a zinc adhesion module, herein described as a "zinc zipper," underscores the criticality of zinc in intercellular adhesion, providing a specific target for chelation and biofilm inhibition. Furthermore, addition of a soluble Aap fragment containing a single intact zinc adhesion module inhibits biofilm formation in a dose-dependent manner, indicating that the G5 domain is indeed a required element for intercellular adhesion in staphylococcal biofilms.

Accordingly, in a first aspect the present invention provides an *in vitro* method for inhibiting formation of a biofilm comprising bacteria, the method comprising contacting the bacteria with an effective amount of at least one zinc chelator, wherein the bacteria comprise at least one zinc adhesion module and wherein the bacteria are selected from the group consisting of *Staphylococcus epidermidis* and *Staphylococcus aureus* and combinations thereof, whereby formation of the biofilm is inhibited; characterized in that the at least one zinc chelator comprises DTPA.

In a second aspect, the present invention provides a method for inhibiting formation of a biofilm on a device, wherein the biofilm comprises bacteria comprising at least one zinc adhesion module and wherein the bacteria are selected from the group consisting of *Staphylococcus epidermidis* and *Staphylococcus aureus* and combinations thereof, the method comprising contacting the device with a solution comprising an effective amount of at least one zinc chelator, whereby formation of a biofilm on the device is inhibited; characterized in that the at least one zinc chelator comprises DTPA.

In a third aspect, the present invention provides a topical pharmaceutical composition comprising a therapeutically effective amount of at least one zinc chelator and at least one pharmaceutically acceptable carrier, for use in a method of inhibiting formation of a biofilm on or in a mammal, wherein the biofilm comprises bacteria comprising at least one zinc adhesion module and wherein the bacteria are selected from the group consisting of *Staphylococcus epidermidis* and *Staphylococcus aureus* and combinations thereof, characterized in that the at least one zinc chelator comprises DTPA.

Also described is a surgical rinse for inhibiting formation of a biofilm comprising bacteria, wherein the bacteria comprise at least one zinc adhesion module, the surgical rinse comprising an effective amount of at least one zinc chelator comprising DTPA.

Also described is a method for inhibiting formation of a biofilm comprising bacteria, the method comprising contacting the bacteria with an effective amount of at least one zinc chelator comprising DTPA, wherein the bacteria are selected from the group consisting of *Staphylococcus aureus,* methicillin resistant *Staphylococcus aureus* (MRSA), *Staphylococcus epidermidis,* methicillin resistant *Staphylococcus epidermidis* (MRSE), *Streptococcus sanguinis,* and *Streptococcus suis,* whereby formation of the biofilm is inhibited.

Also described is a method for inhibiting the formation of a biofilm comprising bacteria, wherein the bacteria comprise at least one zinc adhesion module, the method comprising contacting the bacteria with a composition comprising at least one soluble zinc adhesion module, whereby formation of the biofilm is inhibited.

Also described is a bandage impregnated with a safe and effective amount of at least one zinc chelator comprising DTPA, wherein the bandage inhibits the formation of a biofilm on the skin, wherein the biofilm comprises bacteria comprising at least one zinc adhesion module.

Also described is a personal cleansing composition comprising an effective amount of at least one zinc chelator comprising DTPA, wherein the personal cleansing composition inhibits formation of a biofilm on the skin, wherein the biofilm comprises bacteria comprising at least one zinc adhesion module.

Also described is a hard surface cleaning composition comprising an effective amount of at least one zinc chelator comprising DTPA, wherein the composition inhibits formation of a biofilm on a hard surface, wherein the biofilm comprises bacteria comprising at least one zinc adhesion module.

Also described is a dental rinse for inhibiting formation of a biofilm wherein the biofilm comprises bacteria comprising at least one zinc adhesion module, the dental rinse comprising an effective amount of at least one zinc chelator comprising DTPA.

**Figure 1****.** (A) The five regions of Aap are illustrated: the A-repeat region, the putative globular domain (α/β), the B-repeat region containing 5 to 17 tandem G5 domains, the collagen-like proline/glycine-rich region and a cell wall anchoring motif (LPXTG). The proteolysis site is illustrated with scissors. The domain boundaries of the Brpt1.5 and Brpt2.5 constructs are illustrated, and the histidines are highlighted with arrowheads; (B) Sequence alignment of the terminal G5 domain and C-terminal "half repeat" motif from Aap (SEQ. ID NO. 1) and SasG (SEQ. ID NO. 2) . Identical amino acids are indicated by dashes (-). Blast alignment shows 80% conservation and 65% identity; (C) Far-UV circular dichroism spectrum of Brpt1.5 in 20 mM Tris pH 7.4, 50 mM NaF. Deconvolution of the data reveals predominantly β-sheet and coil secondary structure elements (Table 1); (D) Sedimentation coefficient distribution plot for Brpt1.5 at varying concentrations. Molecular weight estimation indicates Brpt1.5 is monomeric; (E) Representative sedimentation equilibrium data for Brpt1.5, confirming a monomeric state (Table 3). Black lines show the global fits; residuals are shown above.

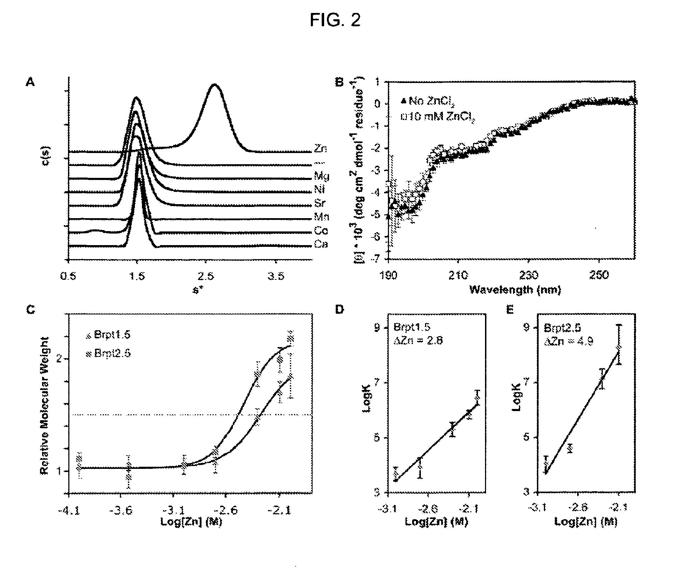

**Figure 2****.** (A) Sedimentation velocity analyses of Brpt1.5 in the presence of divalent cations revealed a Zn²⁺-specific dimerization event. The dashed line label (---) represents cation-free Brpt1.5; (B) Far-UV CD spectrum of Brpt1.5 in the presence (squares) and absence (triangles) of Zn; (C) Sedimentation equilibrium analysis of Zn²⁺-mediated dimerization of Brpt1.5 (triangles) and Brpt2.5 (squares). Brpt2.5 dimerization requires lower [Zn²⁺] (EC₅₀ = 3.7 mM) compared to Brat1.5 (EC₅₀ = 5.4 mM) and shows a steeper slope for the monomer-dimer transition, indicating enhanced cooperativity of Zn²⁺ binding with increasing numbers of tandem G5 domains. Error bars show 95% confidence intervals; (D) Linked equilibrium plot of Zn binding by Brpt1.5. Linear regression analysis of the slope indicates that the number of Zn²⁺ ions taken up upon Brpt1.5 dimerization (i.e., ΔZn²⁺) is approximately three; (E) Linked equilibrium plot of Zn binding by Brpt2.5. Linear regression analysis of the slope indicates that approximately five Zn²⁺ ions are bound upon Brpt2.5 dimerization.

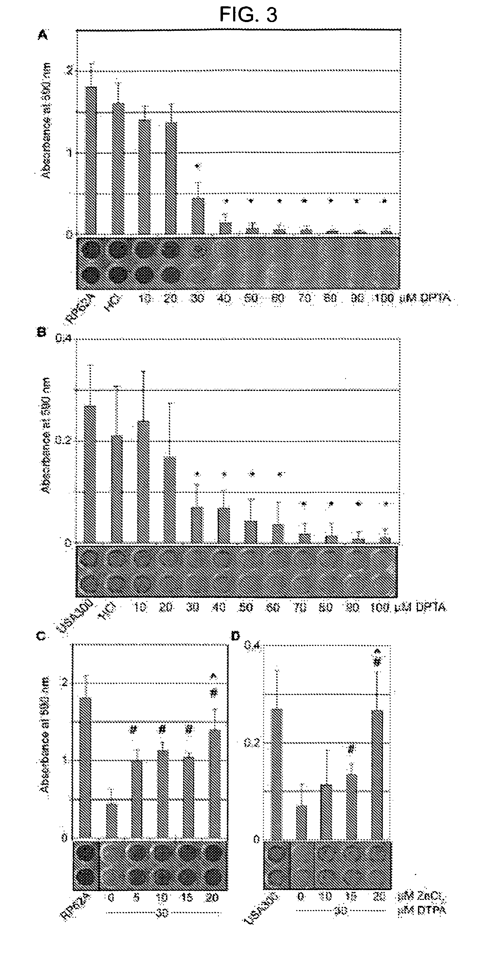

**Figure 3****.** (A) *S. epidermidis* RP62A biofilms formed on polystyrene and were visualized with crystal violet; addition of the Zn²⁺ chelator DTPA (≥30 µM) inhibits biofilm formation. Representative wells have been scanned (bottom). Untreated ("RP62A") and vehicle ("HCl") controls are shown. Error bars show standard deviation (* indicates p<0.05 relative to untreated control; n=3); (B) Methicillin-resistant *S. aureus* USA300 biofilms form on fibronectin-coated plates but are inhibited by addition of DTPA (≥30 µM). (*: p<0.05; n=4); (C) Addition of 5-20 µM ZnCl₂ at a minimal inhibitory dose of DTPA (30 µM) rescues biofilm formation by RP62A. (#: p<0.05 relative to the 0 µM ZnCl₂/30 µM DTPA control; ^: data is statistically indistinguishable from untreated control "RP62A"; n=3); (D) Addition of 15-20 µM ZnCl₂ at a minimal inhibitory dose of DTPA (30 µM) rescues biofilm formation by USA300. (#: p<0.05 compared to 0 µM ZnCl₂/30 µM DTPA; ^: statistically indistinguishable from untreated control; n=4).

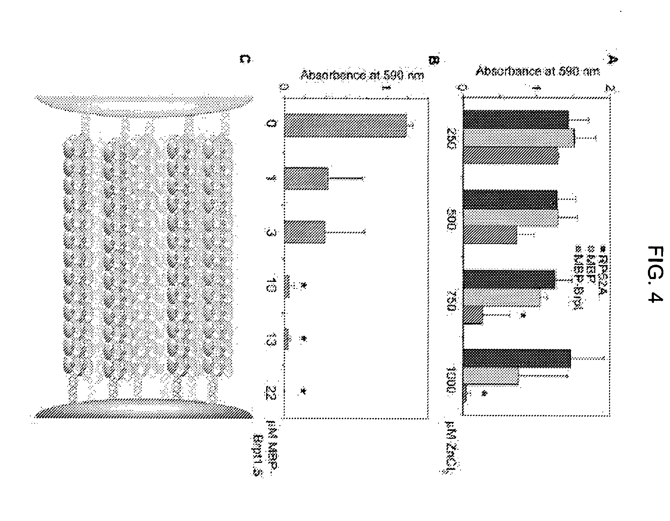

**Figure 4****.** (A) Addition of soluble MBP-Brpt1.5 inhibits RP62A biofilms in a dose-dependent manner in the presence of 0.75-1 mM ZnCl₂. MBP alone was statistically indistinguishable from untreated control at all concentrations tested. (*: p<0.05 relative to RP62A control at the relevant Zn²⁺ concentration; n=3); (B) Dose response of biofilm inhibition by MBP-Brpt1.5 at a fixed 1 mM ZnCl₂ concentration. (*: p<0.0005; n=3); (C) The "zinc zipper" model for intercellular adhesion in staphylococcal biofilms mediated by zinc-dependent self-association of G5 domains.

**Figure 5****.** (A) Aap from *S. epidermidis* strain RP62A. Similar architecture is observed in *S. aureus* proteins SasG and Pls, with the reported number of G5 repeats across Staphylococcal species varying from four to seventeen; (B) "Surface protein from gram positive cocci" from *Streptococcus suis* 89/1591; (C) "LPXTG cell wall surface protein" from *Streptococcus gordonii* str. challis substr. chl; (D) "Putative cell surface protein precursor" from *Corynebacterium urealyticum;* (E). "Conserved hypothetical protein" from *Fingoldia magna;* (F) β-N-acetylhexosaminidase from *Streptococcus pneumoniae.* Similar domain architecture is reported in various *S. pneumoniae* strains; (G) IgA1 protease from *S. pneumoniae.* Similar domain architecture is reported in predicted zinc metalloproteases from *S. pneumoniae, S. gordonii,* and *S. suis,* with up to four repeats of the G5 domain before the N-terminal peptidase domain; (H). VanW vancomycin resistance protein from *Thermosinus carboxydivorans.* Similar proteins are found in *Clostridium botulinum* and *C. difficile, Desulfotomaculum reducens, Caldicellulosiruptor saccharoluticus, Pelotomaculum thermopropionicum, Eubacterium ventriosum, Alkaliphilus sp., Thermonanaerobacter sp., Desulfitobacterium hafniense, Moorella thermoacetica, Carboxydothermus hydreogenoformans,* and *Symbiobacterium thermophilum.*

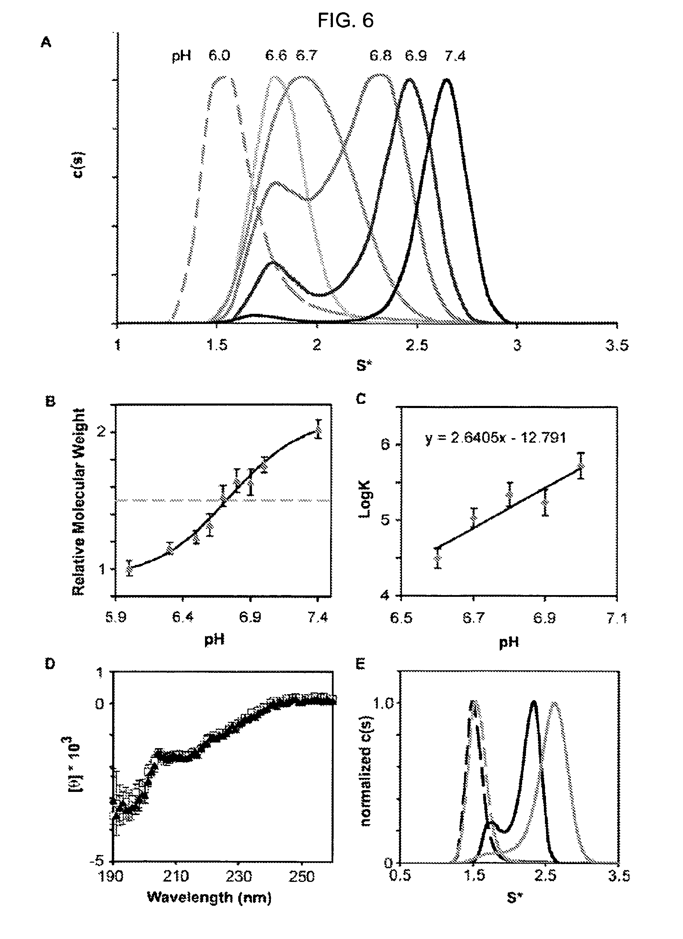

**Figure 6****.** (A) Sedimentation velocity analysis of the Zn²⁺-dependent dimerization of Brpt1.5 as a function of pH in 20 mM Tris, 20 mM MES, 50 mM NaCl, and 10 mM ZnCl₂. Brpt1.5 shifts from dimer at pH 7.4 to monomer at pH 6.0; (B) Sedimentation equilibrium confirms that Brpt1.5 transitions from dimer to monomer as the pH is lowered from 7.4 to 6.0. The apparent pKₐ of this transition is 6.7, which may indicate involvement of histidine residues in Zn²⁺-dependent dimerization; (C) Linked equilibrium analysis of the pH dependence of Brpt1.5 dimerization at 10 mM ZnCl₂. The slope of the logK versus log[H⁺] plot, determined by linear regression, reveals that Brpt1.5 dimerization is linked to the release of approximately 2 or 3 protons; (D) Far-UV CD spectrum of the H75A/H85A Brpt1.5 mutant in the presence (squares) and absence (triangles) of 10 mM ZnCl₂; (E) Sedimentation velocity analysis of wild-type Brpt1.5 (gray) compared to histidine-null mutant H75A/H85A (black). Dashed lines show data collected in the absence of Zn²⁺, and solid lines show data collected at 10 mM ZnCl₂. Partial loss of dimerization is observed in the H75A/H85A mutant, suggesting that one or both histidines as well as other residues are involved in Zn²⁺ coordination.

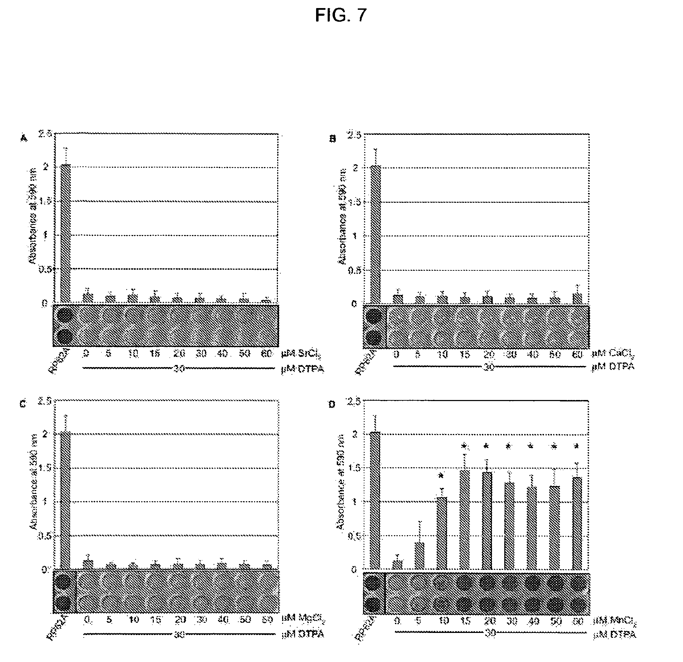

**Figure 7****.** (A) SrCl₂ does not reverse biofilm inhibition by 30 µM DTPA. All data at 30 µM DTPA show statistically significant decreases compared to no-treatment control (RP62A); (B) CaCl₂ does not reverse biofilm inhibition by 30 µM DTPA. All data at 30 µM DTPA show statistically significant decreases compared to no-treatment control (RP62A); (C) MgCl₂ does not reverse biofilm inhibition by 30 µM DTPA. All data at 30 µM DTPA show statistically significant decreases compared to no-treatment control (RP62A); (D) Addition of 10 µM or greater MnCl₂ effected partial rescue of biofilm formation in the presence of 30 µM DTPA. All data at 30 µM DTPA show statistically significant decreases compared to no-treatment control (RP62A). Statistically significant increases over the 0 µM MnCl₂/30 µM DTPA datapoint are marked with an asterisk (*). The levels of MnCl₂ required to rescue biofilm formation were at least 500 times higher than the concentration of Mn in serum (~20 nM), indicating that this is unlikely to be a biologically relevant phenomenon. This result may be due to competition for DTPA and release of chelated zinc. The affinity of Mn for DTPA (log K=15.5) is within approximately three orders of magnitude of the Zn affinity (log K=18.75), whereas the affinities of Ca, Sr, and Mg for DTPA are 8-9 orders of magnitude lower than zinc (log K=10.74, 9.68, and 9.3, respectively).

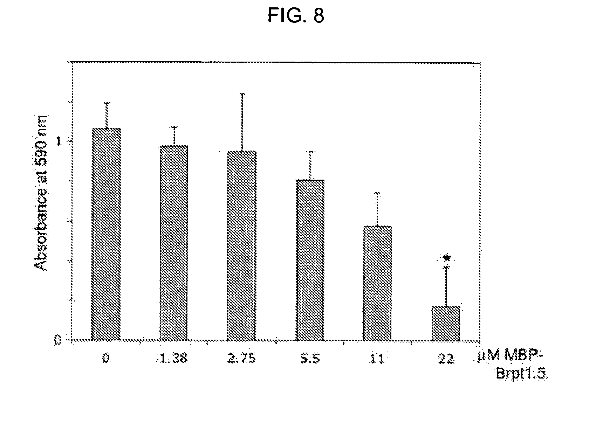

**Figure 8****.** Inhibition of RP62A biofilms by MBP-Brpt1.5 at 750 µM ZnCl₂. Dose response of biofilm inhibition by MBP-Brpt1.5 at a fixed 750 µM ZnCl₂ concentration. Asterisks indicate the level of statistical significance relative to the untreated control.

The following is a list of definitions for terms used herein.

The term "biofilm" refers to matrix-enclosed microbial accretions to biological or non-biological surfaces. Biofilm formation represents a protected mode of growth that allows cells to survive in hostile environments.
The term "biofilm formation" is intended to include the formation, growth, and modification of the bacterial colonies contained with biofilm structures, as well as the synthesis and maintenance of the polysaccharide matrix of the biofilm structures.

The term "zinc adhesion module" refers to a polypeptide fold found in bacterial cell-surface proteins including, but not limited to, the Accumulation-associated protein (Aap) from *Staphylococcus epidermidis.* The zinc adhesion module comprises a polypeptide sequence that includes at least one G5 domain, and optionally additional amino acid sequence.

The term "G5 domain" refers to a polypeptide fold found in a wide variety of extracellular proteins, named after its conserved glycine residues. The G5 domain consists of approximately 80 amino acid residues and is typically found as one to twelve tandem repeats. The G5 domain is the first 80 amino acids of Brpt1.0.

The terms "B-repeat" or "Brpt" refer to a region of the Aap protein having a variable number (5 to 17) of nearly identical 128 amino acid repeats. "Brpt1.5" refers to a B-repeat region terminating in a conserved 79 amino acid "half repeat" motif. "Brpt1.0" refers to an isolated 128 amino acid repeat comprising a G5 domain.

The terms "chelator" or "metal chelator" refer to any substance that is able to remove a metal ion from a solution system by forming a new complex ion that has different chemical properties than those of the original metal ion. The term is further intended to encompass substances that are capable of chelating metal ions, specifically divalent metals.

The term "metal ions" is intended to include any metal ion that is bioavailable, i.e., any metal ion involved in a biochemical reaction or pathway, or any metal ion that is available in the fluid, tissue, or bone of a subject.

The term "zinc chelator" refers to any substance that is able to chelate a zinc (Zn²⁺) ion and thus deplete zinc from aqueous environments.

The term "gram positive bacteria" refers to bacteria having cell walls with high amounts of peptidoglycan. Gram positive bacteria are identified by their tendency to retain crystal violet and stain dark blue or violet in the Gram staining protocol.

The term "gram negative bacteria" refers to bacteria having thinner peptidoglycan layers which do not retain the crystal violet stain in the Gram staining protocol and instead retain the counterstain, typically safranin. Gram negative bacteria stain red or pink in the Gram staining protocol.

The term "implantable medical device" refers to any medical device implanted or inserted in the human body. Such devices can be temporarily or permanently implanted or inserted. An implantable medical device can be, for example, catheters, orthopedic devices, prosthetic devices, vascular stents, urinary stents, pacemakers, implants, or the like.

The term "bathing a device" refers to submerging a device in a solution in order to pre-treat the device, for example, prior to surgical implantation. Bathing a device can also occur after the device has been surgically implanted, for example, by irrigating the surgical site with a sterile solution.

The term "coating a device" refers to pre-treating a device with a composition prior to surgical implantation. Suitable compositions for pre-treating the device may include, for example, solutions, gels, polymer coatings, and the like. A variety of means may be employed to coat a device, such as spraying or submerging the device. The coated device comprises a surface layer having desirable properties conferred by the coating composition. In one embodiment, the coating composition comprises at least one zinc chelator. In another embodiment, the coating composition comprises one or more soluble G5 domains or zinc adhesion modules.

The term "polymer coating" refers to an adherent polymer layer suitable for coating the exterior surface of a device, for example, an implantable medical device. The polymer coating may have intrinsic chelation properties, or may be bonded to one or more chemical or protein-based chelating agents.

The term "mammal" refers to organisms that can suffer from biofilm-associated states. The term includes humans, for example, as well as wild and domesticated animals and livestock, including but not limited to horses, chimpanzees, macaques, pigs, sheep, goats, hamsters, guinea pigs, monkeys, bears, dogs, cats, mice, rabbits, cattle, squirrels, and rats.
The term "pharmaceutical composition" includes preparations suitable for administration to mammals, for example, humans.

The term "topical pharmaceutical composition" refers to pharmaceutical compositions suitable for dermal administration to a mammal. Suitable topical pharmaceutical compositions include, but are not limited to, gels, creams, lotions, ointments, tinctures, sprays, and solids. In one embodiment, a topical pharmaceutical composition of the present invention is applied on the outer surface of the skin or in the vicinity of cuts, abrasions, turf burn injuries, lacerations, burns, or puncture wounds in order to treat, prevent, or inhibit the formation of bacterial biofilms. The term "antimicrobial agent" refers to any substance that kills or prevents the growth of bacteria or other microbes.

The term "antibiotic" refers to a substance that is antagonistic to the growth of microorganisms. Suitable antibiotics may be naturally-occurring, chemically-modified, or synthetically-produced.

The term "surgical rinse" refers to a solution used during surgery to irrigate the site of an implanted medical device, with the intent to prevent initial formation of biofilms in the vicinity of the medical device.

The term "dental rinse" refers to a solution containing one or more zinc chelators used as a mouthwash or rinse to prevent the establishment of oral biofilms that lead to dental caries.

The term "personal cleansing composition" refers to a composition that is used for personal hygiene. Personal cleansing compositions include, but are not limited to, gels, creams, suspensions, colloids, soaps, body washes, shampoos, and the like. In one embodiment, the personal cleansing compositions of the present invention inhibit biofilm-related infections including, but not limited to, community-acquired methicillin-resistant *S. aureus* (CA-MRSA) infection.

The term "hard surface cleaning composition" refers to a composition that is used to clean and/or sanitize a hard or solid surface. In one embodiment, the invention provides a composition that prevents bacterial biofilm growth on hard surfaces including, but not limited to, surgical instruments, storage tanks, pipelines, trays, containers, walls, floors, countertops, locker room floors, benches, lockers, showers, bathrooms, toilets, water filtration units, and the like.

The terms "inhibit," "inhibiting," and "inhibited" as used herein with respect to biofilm formation, refer to the effect of a zinc chelator in disrupting or clearing a biofilm, as well as preventing formation of a biofilm.

The term "effective amount," as used herein with respect to inhibiting biofilm formation, refers to an amount of a zinc chelator or a soluble zinc adhesion module sufficient to achieve the desired inhibitory result.

The term "safe and effective amount" refers to an amount of a zinc chelator or a soluble zinc adhesion module that is effective to inhibit biofilm formation without undue adverse side effects, such as toxicity, irritation, or allergic response, commensurate with a reasonable risk/benefit ration when used in the manner of the invention.

The term "therapeutically effective amount" refers to a sufficient amount of an ingredient to treat disorders, at a reasonable benefit/risk ratio applicable to any medical treatment. It will be understood, however, that the total daily usage of the compositions of the present invention will be decided by the attending physician within the scope of sound medical judgment. The specific therapeutically effective dose level for any particular patient will depend upon a variety of factors including the disorder being treated and the severity of the disorder; activity of the specific chelator employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

### Solution characterization of the terminal G5 domain from Aap

The present invention indicates that homophilic association of Aap and SasG is mediated solely via self-association of their respective G5 domains. The C-terminal G5 domain from the B-repeat region of Aap was expressed both as an isolated domain (called Brpt1.0) and as the single G5 domain followed by the C-terminal 79 amino acid "half-repeat" (called Brpt1.5; Figs. 1A and 1B).

This C-terminal half-repeat is conserved in Aap homo logs, suggesting that it may function as a terminal cap required for the stability of the B-repeat region, as seen with other proteins containing repeating structural motifs. Solution characterization of Brpt1.0 by far-UV circular dichroism (CD) showed a preponderance of random coil with little evidence of regular structural elements. In contrast, Brpt1.5 samples yielded consistent spectra corresponding to 39% β-sheet, 32% coil, 24% turn, and 4% α-helix (Fig. 1C, Table 1).

| Table 1. Circular dichroism secondary structure analyses of Brpt1.5 | | | | |
|---|---|---|---|---|
| | % α-helix | % β-sheet | % Turn | % Coil |
| Brpt1.5 | 4 | 39 | 24 | 32 |
| Brpt1.5 + 10 mM ZnCl₂ | 5 | 38 | 23 | 32 |
| H75A/H85A | 4 | 38 | 23 | 32 |
| H75A/H85A + 10 mM ZnCl₂ | 5 | 40 | 22 | 32 |
| Far-UV CD spectra were collected on samples dialyzed into 20 mM Tris, 50 mM NaF. Secondary structure content was determined using the program CDSSTR on Dichroweb. NRMSD values ranged from 0.039 to 0.043. | | | | |

These data support the role of the half repeat as a structural cap required for stability of the G5 domain. Sedimentation velocity analytical ultracentrifugation (AUC) experiments revealed that Brpt1.5 sedimented as a single elongated species with an estimated molecular weight consistent with a monomer (Fig. 1D, Table 2).

| Table 2. Sedimentation velocity parameters for Brpt1.5 | | |
|---|---|---|
| | s°_{20, w} | f/f₀ |
| Brat1.5 | 1.55 | 2.21 |
| Brpt1.5 + 10 mM ZnCl₂ | 2.46 | 1.66 |
| Values of s°_{20, w} were determined from data at 4 or 5 concentrations. Values of the frictional ratio, f/f₀, were averaged from four or more independent experiments. | | |

No assembly to a higher order species was observed under these conditions, even at high (70 µM) concentrations. Sedimentation equilibrium experiments verified that Brpt1.5 was monomeric under these conditions (Fig. IE, Table 3).

| Table 3. Sedimentation equilibrium parameters for Brpt1.5 and Brpt2.5 plus ZnCl₂. | | | | |
|---|---|---|---|---|
| | M_{sequence} | | Mₑₓₚₑᵣᵢₘₑₙₜₐₗ (95% confidence) | |
| | monomer | dimer | 0 mM Zn | 10 mM Zn |
| Brpt1.5 | 22,284 | 44,568 | 22,075 (19,820-24,259) | 41,213 (38,823-43,536) |
| Brpt2.5 | 35,971 | 71,942 | 36, 987 (34,519-39,380) | 78,541 (76,220-80,863) |
| | K_{D} for monomer-dimer association | | | |
| | 2 mM Zn | 5 mM Zn | 10 mM Zn | |
| Brpt1.5 | 113 µM (62.8-200 µM) | 5.06 µM (3.59-7.08 µM) | 0.353 µM (0.196-0.647 µM) | |
| Brpt2.5 | 25.5 µM (17.8-36.8 µM) | 0.072 µM (0.165-0.0322 µM) | ND* | |
| *ND, Not determined; no detectable monomer was observable under this condition, precluding the determination of an equilibrium constant. | | | | |
| 95% confidence intervals calculated by WinNONLIN. | | | | |
| M_{sequence} is the molecular weight determined by ProtParam from the amino acid sequence. | | | | |
| Mₑₓₚₑᵣᵢₘₑₙₜₐₗ is determined from global analysis of sedimentation equilibrium curves. | | | | |

### Zn²⁺-mediated dimerization of G5 domains

Since many mammalian adhesion proteins including cadherins, integrins, and neurexins require divalent cations for proper function, Brpt1.5 was analyzed by AUC in the presence of Ca²⁺, Sr²⁺ Mg²⁺, Mn²⁺, Ni²⁺, Co²⁺, and Zn²⁺. Interestingly, Brpt1.5 formed dimers only in the presence of Zn²⁺ (Fig. 2A, Tables 2 and 3). Sedimentation equilibrium analyses of Brpt1.5 in the presence of 2, 5, or 10 mM ZnCl₂ revealed monomer-dimer equilibria with dissociation constants of 113 µM, 5.06 µM, and 0.353 µM, respectively (Table 3). CD spectra of Brpt1.5 in the presence or absence of 10 mM ZnCl₂ were similar, indicating that Zn²⁺-mediated Brpt1.5 dimerization occurs as a result of rigid-body association of two G5 domains mediated through coordination of one or more Zn²⁺ ions in *trans* rather than significant Zn²⁺-induced conformational changes (Fig. 2B, Table 1).

In Aap and the related staphylococcal proteins SasG and P1s, G5 domains are found as tandem repeats ranging from 5 to 17 copies. SasG-dependent protein-based biofilm formation in *S. aureus* has been shown to require at least five tandem G5 repeats. *See* Corrigan, Microbiology 153: 2435-46. To analyze the behavior of tandem G5 domains, a Brpt2.5 construct with two G5 domains and the C-terminal cap was expressed.

Sedimentation equilibrium analysis of Brpt2.5 revealed a monomer-dimer equilibrium in the presence of Zn²⁺ as observed for Brpt1.5, but with a modest enhancement in Zn²⁺ affinity for Brpt2.5 compared to Brpt1.5, combined with a steeper transition between monomer and dimer as the repeat length increased (Fig. 2C). To analyze the linked equilibria between dimerization and Zn²⁺ binding, the logarithm of the dimerization association constant was plotted against the logarithm of the free Zn²⁺ concentration to reveal the net number of Zn²⁺ ions participating in the dimerization interface. There are 2-3 Zn²⁺ ions participating in the Brpt1.5 dimer interface, compared to ~5 Zn²⁺ ions for Brpt2.5. This is consistent with a modular arrangement in which each G5 domain contributes to an independent dimer interface (Figs. 2D and 2E).

### Zn²⁺ coordination by the G5 domain

Structural Zn²⁺ ions are most commonly coordinated by cysteine, followed by histidine, aspartate and glutamate. The entire sequence of Aap lacks cysteines; however, there are two histidine residues in the Brpt1.5 construct. Brpt1.5 with 10 mM Zn²⁺ transitions from a dimer at pH 7.4 to a monomer at pH 6.0 (Fig. 6A). Sedimentation equilibrium experiments verified these results; a plot of the relative molecular weight as a function of pH could be fitted to yield an apparent pKₐ of 6.7 (Fig. 6B), consistent with coordination by histidine. Linear regression analysis of the linked equilibria between protonation and dimerization by use of a double-log plot showed a ΔH⁺ value of 2.6, indicating that approximately three ionizable residues are linked to dimerization of Brpt1.5 (Fig. 6C). Site-directed mutagenesis confirmed the involvement of histidines, although other residues such as glutamates or aspartates are likely to contribute as well (Figs. 6D and 6E). Taken together, the CD and AUC results for Brpt1.5 and Brpt2.5 indicate that the G5 domains of Aap self-assemble in a modular fashion upon Zn²⁺ binding. The presence of tandem domain repeats and dependence upon a divalent cation for adhesion are reminiscent of calcium-dependent adhesion by mammalian cadherins.

### Zn²⁺ chelation inhibits staphylococcal biofilm formation

To test the physiological role of Zn²⁺-mediated G5 adhesion in the formation of staphylococcal biofilms, both *S. epidermidis* and *S. aureus* biofilms were grown in culture in the presence or absence of the Zn²⁺ chelator diethylenetriaminepentaacetic acid (DTPA). DTPA was non-toxic at all concentrations tested (up to 100 µM). *S. epidermidis* strain RP62A formed robust biofilms when cultured in media alone, but biofilm growth was inhibited by non-toxic doses of DTPA (≥30 µM) (Fig. 3A). Under the conditions tested, DTPA did not disrupt pre-formed RP62A biofilms.

*S. aureus* strain USA300, which is responsible for recent epidemic outbreaks of community-acquired methicillin-resistant *S. aureus* (MRSA) infections, formed biofilms on fibronectin-coated plates. As observed for *S. epidermidis,* the MRSA biofilms were inhibited by Zn²⁺ chelation (≥30 µM DTPA) (Fig 3B). Surprisingly, *S. epidermidis* biofilm formation could be rescued by the addition of 5-20 µM ZnCl₂, indicating that the effect is Zn²⁺-specific (Fig. 3C). Addition of up to 60 µM CaCl₂, MgCl₂, and SrCl₂ had no effect; MnCl₂ effected partial rescue, but at concentrations nearly 1,000-fold above physiological levels (Fig. 7). Like *S. epidermidis,* the MRSA strain USA300 could be rescued by the addition of 15-20 µM ZnCl₂ (Fig. 3D). Importantly, at the minimal inhibitory concentration of DTPA (30 µM), biofilm growth by both staphylococcal species could be rescued by the addition of 5-20 µM ZnCl₂; these Zn²⁺ concentrations are similar to the physiological range in human plasma (12-16 µM resting concentration).

### Soluble G5 domain inhibits biofilm formation

To determine whether Zn²⁺-dependent biofilm formation is specifically dependent upon G5 domain-mediated self-assembly, soluble Brpt1.5 was added to *S. epidermidis* RP62A biofilms as a maltose-binding protein (MBP) fusion. Because of the higher Zn²⁺ concentrations required to induce dimerization in the short construct compared to full-length Aap, inhibition by MBP-Brpt1.5 was tested at Zn²⁺ concentrations ranging up to 1 mM. RP62A biofilms were inhibited by soluble MBP-Brpt1.5 in the presence of 0.75 and 1 mM ZnCl₂, whereas addition of MBP alone had no effect (Fig. 4A). Titration of MPB-Brpt1.5 at fixed Zn²⁺ concentrations showed that at 1 mM ZnCl₂, addition of 10-22 µM MBP-Brpt1.5 completely abolished biofilm formation (Fig. 4B). Dose-dependent biofilm inhibition by MBP-Brpt1.5 also was observed at 750 µM ZnCl₂ (Fig. 8). Thus, addition of even a single intact zinc adhesion module in a soluble form is capable of inhibiting biofilm formation, highlighting the role of this domain as a Zn²⁺-dependent adhesion module in staphylococcal biofilms.

The G5 domain, found in Aap, SasG, and related staphylococcal proteins such as Pls, mediates Zn²⁺-dependent staphylococcal biofilm formation. Multiple self-association events between stretches of tandem G5 domains in opposing Aap molecules would lead to an extensive adhesive contact between two cells, a so-called "zinc zipper" (Fig. 4C). Aap was originally identified as a protein required for the formation of *S. epidermidis* microcolonies. Subsequently, Aap and SasG were shown to undergo a proteolysis event near the beginning of the B-repeat region containing the tandem G5 domains, which leads to formation of protein-based staphylococcal biofilms. The present invention indicates that the same mechanism for intercellular adhesion based upon Zn²⁺-dependent G5 self-association is responsible for early and late stages of biofilm formation. Zn²⁺-dependent intercellular adhesion in biofilms mediated by G5 domains has likely evolved as a defense mechanism against immune cell action. Zn²⁺ levels that are known to increase cytokine release and boost host immune responses are sufficient to promote staphylococcal biofilm formation (5-20 µM Zn²⁺). Mast cells, basophils, and eosinophils contain high levels of Zn²⁺ in secretory granules that is released upon degranulation.

Given the global emergence of staphylococcal biofilm-related infections, improved methods for prevention are needed. Targeting G5 self-association either by Zn²⁺ chelation or direct inhibition with small molecule compounds provides a new therapeutic avenue for combating the formation of biofilms by a broad range of bacterial species, including epidemic MRSA strains. The application to MRSA infections is of particular interest, given that MRSA was recently reported to cause more deaths per year than AIDS. King, M.D. et al, Emergence of community-acquired methicillin-resistant Staphylococcus aureus USA 300 clone as the predominant cause of skin and soft-tissue infections, Ann. Intern. Med. 144:309-17 (2006). Finally, genes encoding surface proteins with one or more tandem G5 domains are found in a vast number of bacterial species, indicating that the zinc zipper mechanism for intercellular adhesion provides a target for inhibition of biofilm formation across multiple bacterial species, in both medical and industrial applications.

### Biofilm-forming bacteria

For each of the subsequent embodiments of the invention, the biofilm-forming bacteria comprise at least one zinc adhesion module, such that contact with one or more zinc chelators or one or more soluble zinc adhesion modules inhibits formation of a biofilm. For each embodiment, the bacteria comprising at least one zinc adhesion module may be selected from the group consisting *Staphylococcus epidermidis* and *Staphylococcus aureus* and combinations thereof.

Other bacteria that form biofilms include Acidothermus cellulyticus, Actinomyces odontolyticus, Alkaliphilus metalliredigens, Alkaliphilus oremlandii, Arthrobacter aurescens, Bacillus amyloliquefaciens, Bacillus clausii, Bacillus halodurans, Bacillus licheniformis, Bacillus pumilus, Bacillus subtilis, Bifidobacterium adolescentis, Bifidiobacterium longum, Caldicellulosiruptor saccharolyticus, Carboxydothermus hydrogenoformans, Clostridium acetobutylicum, Clostridium beijerinckii, Clostridium botulinum, Clostridium cellulolyticum, Clostridium difficile, Clostridium kluyveri, Clostridium leptum, Clostridium novyi, Clostridium perfringens, Clostridium tetani, Clostridium thermocellum, Corynebacterium diphtheriae, Corynebacterium efficiens, Corynebacterium glutamicum, Corynebacterium jeikeium, Corynebacterium urealyticum, Desulfitobacterium hafniense, Desulfotomaculum reducens, Eubacterium ventriosum, Exiguobacterium sibiricum, Fingoldia magna, Geobacillus kaustophilus, Geobacillus thermodenitrificans, Janibacter sp., Kineococcus radiotolerans, Lactobacillus fermentum, Listeria monocytogenes, Listeria innocua, Listeria welshimeri, Moorella thermoacetica, Mycobacterium avium, Mycobacterium bovis, Mycobacterium gilvum, Mycobacterium leprae, Mycobacterium paratuberculosis, Mycobacterium smegmatis, Mycobacterium tuberculosis, Mycobacterium ulcerans, Mycobacterium vanbaalenii, Nocardioides sp., Nocardia farcinica, Oceanobacillus iheyensis, Pelotomaculum thermopropionicum, Rhodococcus sp., Saccharopolyspora erythraea, coagulase-negative Staphylococcus species, Staphylococcus aureus, methicillin resistant Staphylococcus aureus (MRSA), Staphylococcus epidermidis, methicillin resistant Staphylococcus epidermidis (MRSE), Streptococcus agalactiae, Streptococcus gordonii, Streptococcus mitis, Streptococcus oralis, Streptococcus pneumoniae, Streptococcus sanguinis, Streptococcus suis, Streptomyces avermitilis, Streptomyces coelicolor, Thermoanaerobacter ethanolicus, Thermoanaerobacter tengcongensis, and combinations thereof.

Even more specifically, the bacteria may be selected from the group consisting of *Corynebacterium urealyticum, Fingoldia magna, Staphylococcus aureus,* methicillin resistant *Staphylococcus aureus* (MRSA), *Staphylococcus epidermidis,* methicillin resistant *Staphylococcus epidermidis* (MRSE), *Streptococcus gordonii, Streptococcus pneumonia, Streptococcus sanguinis, Streptococcus suis,* and combinations thereof.

More specifically still, the bacteria may be selected from the group consisting of *Staphylococcus aureus,* methicillin resistant *Staphylococcus aureus* (MRSA), *Staphylococcus epidermidis,* methicillin resistant *Staphylococcus epidermidis* (MRSE), and combinations thereof.

It has also been discovered that certain gram negative bacteria comprise at least one zinc adhesion module. For example, gram negative bacteria comprising a zinc adhesion module include, but are not limited to, *Bacteroides capillosus, Symbiobacterium thermophilum, Syntrophomonas wolfei, Thermosinus carboxydivorans.* Accordingly, it is envisaged that the invention may also be applied to gram negative bacteria having at least one zinc adhesion module.

### Zinc chelators

One skilled in the art will appreciate the variety of substances capable of chelating zinc metal ions. The zinc chelator of the present invention comprises 1,3-diaminopropane-N,N,N',N'-tetraacetic acid (DTPA). Examples of zinc chelators include ethylenediaminetetra-acetic acid (EDTA), 1,3-diaminopropane-N,N,N',N'-tetraacetic acid (DTPA), N,N,N',N'-tetrakis(2-pyrdiylmethyl) ethylenediamine (TPEN), 1,10-phenanthroline, clioquinol, diethyldithiocarbamate (DEDTC), 2,3-dimercapto-1-propanesulfonic acid (DMPS), ethylenediamine - N,N'- diacetic-N,N'-di-B-propionic acid (EDPA), 1,2-dimethyl-3-hydroxy-4-pyridinone (DMHP), 1,2-diethyl-3-hydroxy-4-pyridinone (DEHP), ethylmaltol (EM), 4-(6-Methoxy-8-quinaldinyl-aminosulfonyl)benzoic acid potassium salt (TFLZn), dithiozone, N-(6-methoxy-8-quinolyl)-para-toluenesulfonamide (TSQ), carnosine, deferasirox, trans-1,2-cyclohexane-diamine-N,N,N',N'-tetraacetic acid (CyDTA), dihydroxyethylglycine (DHEG), 1,3-diamino-2-hydroxypropane-N,N,N',N'-tetraacetic (DTPA-OH), ethylenediamine - N,N'-diacetic acid (EDDA), ethylenediamine-N,N'-dipropionic acid (EDDP), ethylenediamine-N,N'-bis(methylphosphonic) acid (EDDPO), N-hydroxy-ethylenediamine-N,N',N'-triacetic acid (EDTA-OH), ethylenediaminetetra(methylenephosphonic) acid (EDTPO), N,N'-bis(2-hydroxybenzyl)ethylenediamine-N,N'-diacetic acid (HBED), hexamethylene-1,6-diaminetetraacetic acid (HDTA), hydroxyethyliminodiacetic acid (HIDA), iminodiacetic acid (IDA), Methyl-EDTA, nitrilotriacetic acid (NTA), nitrilotripropionic acid (NTP), Nitrilotrimethylenphosphonic acid (NTPO), 7,19,30-trioxa-1,4,10,13,16,22,27,33- octaazabicyclo[11,11,11] pentatriacontane (O-Bistren), triethylenetetraaminehexaacetic acid (TTHA), ethyleneglycol bis(2-Aminoethyl ether)-N,N,N',N'-tetraacetic acid (EGTA), dimercaptosuccinic acid (DMSA), deferoxamine, dimercaprol, zinc citrate, combination of bismuth and citrate, penicilamine, succimer, Etidronate, ethylenediamine-di (O-hydroxyphenylacetic acid) (EDDHA), trans-1,2-cyclohexanediaminetetraacetic acid (CDTA), N-(2-hydroxyethyl) ethylenedinitrilotriacetic acid (HEDTA), N-(2-hydroxyethyl) iminodiacetic acid (HEIDA), calprotectin, zinc fingers, lactoferrin, ovotransferrin, conalbumin, and combinations thereof.

The invention relates to a method for inhibiting formation of a biofilm comprising bacteria, the method comprising contacting the bacteria with an effective amount of at least one zinc chelator comprising DTPA, wherein the bacteria comprise at least one zinc adhesion module and wherein formation of the biofilm is inhibited.

The at least one zinc chelator is placed in contact with the bacteria within an environment that supports bacterial adhesion and therefore potential establishment of biofilm formation. Bacterial zinc deprivation at the site of adhesion prevents zinc adhesion module interaction and, accordingly, protein-dependent biofilm formation. Zinc chelators, however, are not necessarily bactericidal and may not directly kill cells, but instead prevent establishment of infection.

A variety of zinc chelators are known in the art. Determining the appropriate concentration of the zinc chelator, based on its properties and the targeted bacterial biofilm(s), is within the purview of the skilled artisan. For example, appropriate concentrations of DTPA for use against the bacteria *Staphylococcus aureus,* methicillin resistant *Staphylococcus aureus, Staphylococcus epidermidis,* methicillin resistant *Staphylococcus epidermidis, Streptococcus gordonii, Streptococcus pneumoniae, Streptococcus sanguinis,* and *Streptococcus suis* may range from about 10µM to about 1000µM, from about 10µM to about 800µM, from about 10µM to about 700µM, from about 20µM to about 640µM, from about 20µM to about 500µM, from about 20µM to about 400µM, from about 20µM to about 320µM, from about 20µM to about 200µM, or from about 20µM to about 160µM.

The invention also relates to a method for inhibiting formation of a biofilm on a device, wherein the biofilm comprises bacteria comprising at least one zinc adhesion module, the method comprising contacting the device with a solution comprising an effective amount of at least one zinc chelator comprising DTPA, wherein formation of a biofilm on the device is inhibited.

In one embodiment of the invention, the device is an implantable medical device. One skilled in the art will appreciate that any medical device implanted or inserted into the body is suitable for use in the present embodiment. For example, suitable implantable medical devices include, but are not limited to, pacemakers, heart valves, replacement joints, catheters, catheter access ports, dialysis tubing, gastric bands, shunts, screw plates, artificial spinal disc replacements, internal implantable defibrillators, cardiac resynchronization therapy devices, implantable cardiac monitors, mitral valve ring repair devices, left ventricular assist devices (LVADs), artificial hearts, implantable infusion pumps, implantable insulin pumps, stents, implantable neurostimulators, maxillofacial implants, dental implants, and the like.

The biofilm-forming bacteria of the embodiment comprise at least one zinc adhesion module, such that contact with one or more zinc chelators comprising DTPA inhibits formation of a biofilm on the device.

Determining the appropriate concentration of the zinc chelator, based on its properties and the bacterial biofilm at issue, is well within the purview of the skilled artisan. For example, the concentration of the chelator can be from about 20 µM to about 3 mM. For example, appropriate concentrations of DTPA for use in the present method with *S. epidermidis* RP62A may range from about 20 µM to about 2.6 mM, or more specifically from about 20 µM to about 80 µM, or still more specifically from about 30 µM to about 80 µM; with *S. aureus* USA300, from about 80 µM to about 1300 µM, or more specifically from about 80 µM to about 1600 µM; with *S. aureus* SA113, from about 10 µM to about 160 µM, or more specifically from about 10 µM to about 30 µM; and with *S*. *aureus* COL, from about 10 µM to about 160 µM, or more specifically from about 10 µM to about 30 µM.

In one embodiment, the device is bathed or coated in a solution comprising an effective amount of at least one zinc chelator comprising DTPA. For example, the device may be dipped in a solution comprising an effective amount of a zinc chelator and optionally allowed to dry. In another aspect of the embodiment, the device may be sprayed with a solution comprising a zinc chelator comprising DTPA and optionally allowed to dry. In a surgical setting, the present method could be used, for example, to bathe a medical device prior to implantation. Alternatively, a medical device could be coated with a solution comprising a zinc chelator prior to implantation.

The solution comprising the zinc chelator comprising DTPA can be in any form which permits application of the solution to the particular device. In a specific embodiment, the solution is a gel comprising at least one zinc chelator comprising DTPA. In another specific embodiment, the solution is a polymer coating comprising at least one zinc chelator. Optionally, the solution may be composed such that an effective amount of a zinc chelator is released gradually, providing for inhibition of biofilm formation over a period of time.

The invention further relates to a topical pharmaceutical composition for use in a method of inhibiting formation of a biofilm in or on a mammal is provided, wherein the biofilm comprises bacteria comprising at least one zinc adhesion module, the pharmaceutical composition comprising a therapeutically effective amount of at least one zinc chelator comprising DTPA and at least one pharmaceutically acceptable carrier.

The phrase "pharmaceutically acceptable carrier" is art recognized and includes a pharmaceutically acceptable material, composition or vehicle, suitable for administering compositions of the present invention to mammals. Each carrier must be "acceptable" in the sense of being compatible with the other ingredients of the formulation and not injurious to the patient.

The compositions of this invention can be administered topically to a subject, e.g., by the direct laying on or spreading of the composition on the epidermal or epithelial tissue of the subject. Such compositions include, for example, lotions, creams, solutions, gels, sprays, ointments, and solids. These topical compositions preferably comprise a therapeutically effective amount, usually at least about 20 µM, and specifically from about 40 µM to about 2 mM, of a zinc chelator comprising DTPA. Suitable carriers for topical administration preferably remain in place on the skin as a continuous film, and resist being removed by perspiration or immersion in water. Generally, the carrier is organic in nature and capable of having dispersed or dissolved therein at least one zinc chelator comprising DTPA. The carrier may include pharmaceutically-acceptable emollients, emulsifiers, thickening agents, solvents, and the like.

The amount of zinc chelator comprising DTPA to be topically administered depends upon such factors as skin sensitivity, type and location of the tissue to be treated, the composition and carrier to be administered, the particular zinc chelator comprising DTPA to be administered, as well as the particular bacterial biofilm to be inhibited.

The compositions of the invention may further include additional drugs or excipients as appropriate for the indication. In one embodiment, the pharmaceutical composition further comprises a therapeutically effective amount of at least one antimicrobial agent. In a more specific embodiment, the antimicrobial agent is an antibiotic.

A surgical rinse may be used for inhibiting the formation of a biofilm, wherein the biofilm comprises bacteria comprising at least one zinc adhesion module, and wherein the surgical rinse comprises an effective amount of at least one zinc chelator comprising DTPA. The surgical rinse may be, for example, a buffered saline solution or a Ringer's solution. The chelator may be DTPA and the concentration can be from about 20 µM to about 2mM. A surgical rinse of this kind may be applied before, during, or after surgery and may be aspirated from the surgical area or left on the surgical area to inhibit biofilm formation. The bandage may be impregnated with a chelating polymer composition.

Also described is a method for inhibiting formation of a biofilm comprising bacteria, the method comprising contacting the bacteria with an effective amount of at least one zinc chelator comprising DTPA, wherein the bacteria are selected from the group consisting of *Staphylococcus aureus,* methicillin resistant *Staphylococcus aureus* (MRSA), *Staphylococcus epidermidis,* methicillin resistant *Staphylococcus epidermidis* (MRSE), *Streptococcus sanguinis,* and *Streptococcus suis,* whereby formation of the biofilm is inhibited.

Also described is a method for inhibiting the formation of a biofilm comprising bacteria wherein the bacteria comprise at least one zinc adhesion module, the method comprising contacting the bacteria with a composition comprising at least one soluble zinc adhesion module, wherein formation of the biofilm is inhibited. The composition may be, for example, a spray, lotion, solution, gel, cream, ointment, surgical rinse, or dental rinse. The composition comprising at least one soluble zinc adhesion module may be a device-soaking solution, a personal cleaning composition, or a hard surface cleaning composition prepared with recombinant, purified soluble zinc adhesion modules. The composition may comprises about 5 µM to about 50 µM of a protein construct comprising one or two zinc adhesion modules, or about 10 nM to about 10 µM of a protein construct containing three to 17 zinc adhesion modules.

A bandage impregnated with a safe and effective amount of at least one zinc chelator comprising DTPA may be used, wherein the bandage inhibits the formation of a biofilm on the skin, wherein the biofilm comprises bacteria comprising at least one zinc adhesion module. The bandage may be suitable for use in patients with cuts, burns, turf burns, abrasions, lacerations, puncture wounds, regions of bacterial infection such as boils and pustules, and the like. The chelator may be DTPA and the concentration of DTPA in the solution impregnating the bandage may be from about 20 µM to about 2 mM.

A personal cleansing composition comprising an effective amount of at least one zinc chelator comprising DTPA is also envisaged, wherein the personal cleansing composition inhibits formation of a biofilm on the skin, wherein the biofilm comprises bacteria comprising at least one zinc adhesion module. Suitable personal cleansing compositions include, but are not limited to, surgical scrubs, shower gels, body washes, soaps, and the like. The chelator may be DTPA and the concentration of DTPA in the personal cleansing composition may be from about 20 µM to about 2 mM. The personal cleansing composition may be applied as a part of a personal hygiene routine. Personal cleansing compositions of this type may be suitable for use by a variety of individuals, including, for example, people recovering from MRSA or MRSE infections, athletes using team locker rooms, and healthcare professionals.

A hard surface cleaning composition comprising an effective amount of at least one zinc chelator comprising DTPA is also envisaged, wherein the composition inhibits formation of a biofilm on a hard surface, wherein the biofilm comprises bacteria comprising at least one zinc adhesion module. Such a hard surface cleaning composition has a variety of useful applications, including use in industrial applications as well as medical, veterinary, or livestock environments. For example, hard surface cleaners of this type are useful in the cleaning and treating of pipeline systems, cooling water systems in power plants, refineries, chemical plants, air conditioning systems, storage tanks, trays, containers, walls, floors, countertops, locker room floors, benches, lockers, showers, bathrooms, toilets, water filtration units, and the like, as part of a standard cleaning routine. The chelator may be DTPA and the concentration of chelator in the composition may be from about 20 µM to about 20 mM. Optionally, the at least one zinc chelator comprising DTPA is present in the composition in combination with one or more other cleaning agents, such as detergents, surfactants, alcohols, hydrochloric or muriatic acid, acetic acid, sodium or potassium hydroxide, ammonia, and the like.

A dental rinse is also envisaged for inhibiting formation of a biofilm wherein the biofilm comprises bacteria comprising at least one zinc adhesion module, the dental rinse comprising an effective amount of at least one zinc chelator comprising DTPA.

### EXAMPLES

The following examples are given by way of illustration only and are not intended to limit the scope of the present invention.

### EXAMPLE 1

### Preparation of DTPA zinc chelator stock solutions

A composition according to the present invention comprising aqueous DTPA in the amounts indicated was prepared:

A 100 mM stock solution was prepared by dissolving solid DTPA in 500 mM HCl and incubating at room temperature with agitation (rocking) for 2 hrs until all powder dissolved. This concentrated stock solution was used to prepare 8-10X stocks to use for the assay, by diluting concentrated DTPA into filtered water to give final concentrations 200, 400, 800, 1600, 3200, 6400, 13000, 26000 µM. All chelator solutions were filter-sterilized by a 0.2 µm nitrocellulose filter prior to use in the biofilm assay.

### COMPARATIVE EXAMPLE 2

### Preparation of EDTA zinc chelator stock solutions

A composition according to the present invention comprising aqueous EDTA in the amounts indicated was prepared:

A concentrated 0.5 M stock solution was prepared by dissolving solid EDTA in filtered water and titrating in 10 M NaOH dropwise until a pH 8.8 was reached. This concentrated stock solution was used to prepare 8-10X stocks for the assay by diluting into filtered water and filter sterilizing, as described in Example 1.

### COMPARATIVE EXAMPLE 3

### Preparation of EDDA zinc chelator stock solutions

A composition according to the present invention comprising aqueous EDDA in the amounts indicated was prepared:

A concentrated 100 mM stock was prepared by dissolving solid EDDA in 1N NaOH and titrating in 12 M HCl dropwise until a pH of 8.8 was reached. This concentrated stock solution was used to prepare 8-10X stocks for assay by diluting into filtered water and filter sterilizing, as described in Example 1.

### COMPARATIVE EXAMPLE 4

### Preparation of DEDTC zinc chelator stock solutions

A composition according to the present invention comprising aqueous DEDTC in the amounts indicated was prepared:

A concentrated 100 mM stock was prepared by dissolving solid DEDTC in filtered water. This concentrated stock solution was used to prepare 8-10X stocks for assay by diluting into filtered water and filter sterilizing, as described in Example 1.

### COMPARATIVE EXAMPLE 5

### Preparation of TPEN zinc chelator stock solutions

A composition according to the present invention comprising aqueous TPEN in the amounts indicated was prepared:

A concentrated 100 mM stock was prepared by dissolving solid TPEN in 100% ethanol. This concentrated stock solution was used to prepare 8-10X stocks for assay by diluting into 100% ethanol and filter sterilizing, as described in Example 1.

### COMPARATIVE EXAMPLE 6

### Preparation of 1,10 - phenanthroline zinc chelator stock solutions

A composition according to the present invention comprising aqueous 1,10 - phenanthroline in the amounts indicated was prepared:

A concentrated 100 mM stock was prepared by dissolving solid 1,10 - phenanthroline in 100 mM HCl. This concentrated stock solution was used to prepare 8-10X stocks for assay by diluting into filtered water and filter sterilizing, as described in Example 1.

### COMPARATIVE EXAMPLE 7

### Preparation of crystal violet stock solution

A composition according to the present invention comprising aqueous crystal violet in the amounts indicated was prepared:

A stock solution was prepared by dissolving 1 gram of solid crystal violet in 1 liter of filtered water, and passing the solution through a 0.45 µm nitrocellulose filter.

### EXAMPLE 8

### Effectiveness of zinc chelators against biofilm formation by

### methicillin-resistant Staphylococcus epidermidis strain RP62a

*S. epidermidis* strain RP62a (ATCC 35984) was obtained as a glycerol stock from ATCC. 3 ml overnight cultures were grown in tryptic soy broth (TSB, BD Biomedical), shaking at 37 °C, and cultures were diluted 1:200 in TSB for use in the biofilm assay.

Each chelator (prepared in Examples 1-6) was added directly to a 96 well microtiter plate in a series of dilutions. 20 µl of each stock solution (200, 400, 800, 1600, 3200, 6400, 13000, or 26000 µM chelator) was added to each well. 200 µl of the 1:200 dilution of RP62a bacterial culture (as previously specified) was added to each well. Controls with bacterial culture media only and bacterial culture media plus vehicle controls only (i.e., final concentrations 10mM HCl, 10% ethanol, or 10mM NaOH) were added to wells alone without chelator. The 96 well plates were then incubated 18-24 hours statically at 37 °C. Media was aspirated by pipet and wells were washed twice with filtered water. The plates were dried by inversion for 1 hour, and stained with 0.1% crystal violet for 15 minutes. Crystal violet was then aspirated by pipet and washed once with filtered water. The plates were then read on a spectrophotometer at a wavelength of 570 nm. An absorbance greater than 0.1 indicates biofilm formation.

For RP62a, biofilms were diminished but not cleared by 20 µM DTPA, 40 µM EDTA, 80 µM 1,10-phenanthroline, 20 µM DEDTC, and 80 µM EDDA. RP62a biofilms were inhibited (i.e., absorbance of 0.1 or less) by 40 µM DTPA, 20 µM TPEN, 320 µM EDTA, 640 µM 1,10-phenanthroline, and 1.3 mM DEDTC.

### EXAMPLE 9

### Effectiveness of zinc chelators against biofilm formation by Staphylococcus aureus strain Rosenbach SA113

*S. aureus* strain Rosenbach SA113 (ATCC 35556) was obtained as a glycerol stock from ATCC. 3 ml overnight cultures were grown in blood-heart infusion broth (BHI, BD biomedical) shaking at 37 °C, where cultures were diluted 1:50 for use in the biofilm assay.

2% glucose (20 µl from a 20% glucose stock) was added to each well prior to the addition of bacterial culture to promote biofilm formation. In addition, 96 well microtiter plates were pre-treated with fetal bovine serum (FBS) at 4 °C for at least 24 hours prior to use. The FBS was aspirated prior to addition of chelators and glucose for the assay. Each chelator (prepared in Examples 1-6) was added directly to a 96 well microtiter plate in a series of dilutions. 20 µl of each stock solution (200, 400, 800, 1600, 3200, 6400, 13000, or 26000 µM chelator) was added to each well. 200 µl of the 1:50 dilution of SA113 bacterial culture (as previously specified) was added to each well. Controls with bacterial culture media only and bacterial culture media plus vehicle controls only (i.e., final concentrations 10mM HCl, 10% ethanol, or 10mM NaOH) were added to wells alone without chelator. The 96 well plates were then incubated 36-40 hours statically at 37 °C. Media was aspirated by pipet and wells were washed twice with filtered water. The plates were dried by inversion for 1 hour, and stained with 0.1% crystal violet for 15 minutes. Crystal violet was then aspirated by pipet and washed once with filtered water. The plates were then read on a spectrophotometer at a wavelength of 570 nm. An absorbance greater than 0.1 indicates biofilm formation.

For SA113, biofilms were diminished but not cleared by 20 µM TPEN, 320 µM EDTA, 320 µM 1,10-phenanthroline, 80 µM DEDTC, and 20 µM EDDA. SA113 biofilms were inhibited (i.e., absorbance of 0.1 or less) by 20 µM DTPA, 40 µM TPEN, 1.3 mM 1,10-phenanthroline, 80 µM DEDTC, and 640 µM EDDA.

### EXAMPLE 10

### Effectiveness of zinc chelators against biofilm formation by Staphylococcus aureus strain COL

*S. aureus* strain COL was obtained directly from Brian Wilkinson (Illinois State University) who has maintained the culture as a frozen stock since 1976. 3 ml overnight cultures were grown in blood-heart infusion broth (BHI) shaking at 37 °C, where cultures were diluted 1:25 for use in the biofilm assay.

2% sucrose (20 µl from a 20% sucrose stock) was added to each well prior to the addition of the bacterial culture to promote biofilm formation. In addition, 96 well microtiter plates were pre-treated with fetal bovine serum (FBS) at 4 °C for at least 24 hours prior to use. The FBS was aspirated prior to addition of chelators and sucrose for the assay. Each chelator (prepared in Examples 1-6) was added directly to a 96 well microtiter plate in a series of dilutions. 20 µl of each stock solution (200, 400, 800, 1600, 3200, 6400, 13000, or 26000 µM chelator) was added to each well. 200 µl of the 1:25 dilution of COL bacterial culture (as previously specified) was added to each well. Controls with bacterial culture media only and bacterial culture media plus vehicle controls only (i.e, final concentrations 10 mM HCl, 10% ethanol, and 10 mM NaOH) were added to wells alone without chelator. The 96 well plates were then incubated 36-40 hours statically at 37 °C. Media was aspirated by pipet and wells were washed twice with filtered water. The plates were dried by inversion for 1 hour, and stained with 0.1% crystal violet for 15 minutes. Crystal violet was then aspirated by pipet and washed once with filtered water. The plates were then read on a spectrophotometer at a wavelength of 570 nm. An absorbance greater than 0.1 indicates biofilm formation.

For COL, biofilms were diminished but not cleared by 20 µM EDTA, 80 µM 1,10-phenanthroline, 20 µM DEDTC, and 160 µM EDDA. COL biofilms were inhibited (i.e., absorbance of 0.1 or less) by concentrations greater than 20 µM DTPA, 20 µM TPEN, 160 µM EDTA, 320 µM 1,10-phenanthroline, and 80 µM DEDTC. 20.

### EXAMPLE 11

### Effectiveness of zinc chelators against biofilm formation by methicillin-resistant Staphylococcus aureus strain USA300

*S. aureus* strain USA300 was obtained from Daniel Hassett (University of Cincinnati) as a glycerol stock. 3 ml overnight cultures were grown in blood-heart infusion broth (BHI) shaking at 37 °C, where cultures were diluted 1:25 for use in the biofilm assay.
2% glucose (20 ul from a 20% glucose stock) was added to each well prior to the addition of bacterial culture to promote biofilm formation. In addition, 96 well microtiter plates were pre-treated with human fibronectin (BD biomedical purchased from Fisher scientific). The fibronectin was aspirated prior to addition of chelators and glucose for the assay. Each chelator (prepared in Examples 1-6) was added directly to a 96 well microtiter plate in a series of dilutions. 20 µl of each stock solution (200, 400, 800, 1600, 3200, 6400, 13000, or 26000 µM chelator) was added to each well. 200 µl of the 1:25 dilution of USA300 bacterial culture dilution (as previously specified) were added to each well. Controls with bacterial culture media only and bacterial culture media plus vehicle controls only (final concentrations 10 mM HCl, 10% ethanol, and 10 mM NaOH) were added to wells alone without chelator. The 96 well plates were then incubated 36-40 hours statically at 37 °C. Media was aspirated by pipet and wells were washed twice with filtered water. The plates were dried by inversion for 1 hour, and stained with 0.1% crystal violet for 15 minutes. Crystal violet was then aspirated by pipet and washed once with filtered water. The plates were then read on a spectrophotometer at a wavelength of 570 nm. An absorbance greater than 0.1 indicates biofilm formation.

For USA300, biofilms were diminished but not cleared by 20 µM DTPA, 20 µM TPEN, 1.3 mM EDTA, and 160 µM 1,10-phenanthroline. USA300 biofilms were inhibited (i.e., absorbance of 0.1 or less) by concentrations greater than 20 µM DTPA, 20 µM TPEN, 2.6 mM EDTA, and 1.3 mM 1,10-phenanthroline.

### EXAMPLE 12

A prosthetic knee replacement is prepared prior to surgery by bathing the device for 60 minutes in a 2 mM sterile DTPA solution. The solution is an aqueous solution containing 10 mM HCl, a vehicle used to solubilized DTPA. When the surgeon is ready to insert the prosthetic knee replacement, the device is removed from the sterile DTPA solution and implanted into the patient. Treatment with the DTPA solution inhibits biofilm formation on the prosthetic device.

### EXAMPLE 13

During the surgical installation of an implantable medical device, the vicinity of the surgical operation is rinsed with a sterile buffered saline solution containing 100 µM DTPA. Upon the conclusion of the surgery, the sterile solution is aspirated from the surgical site. Rinsing with the DTPA solution inhibits biofilm formation on the implantable device by commensal or environmental bacteria that gain access to the implantation site during surgery.

### EXAMPLE 14

A topical pharmaceutical cream containing 100 µM DTPA is prepared. This composition is applied to the surface of the skin after a turf burn injury. Treatment with the topical pharmaceutical cream inhibits biofilm formation upon infection of the injured area with methicillin resistant *Staphylococcus aureus.*

### EXAMPLE 15

A bandage is impregnated with a pharmaceutical gel containing 100 µM DTPA. The bandage is applied to the surface of the skin over a laceration. Treatment with the bandage inhibits biofilm formation upon infection of the injured area with *Staphylococcus epidermidis.*

### EXAMPLE 16

A personal cleaning composition in the form of a shower gel containing 1 mM DTPA is prepared. The personal cleaning composition is applied as a part of a personal hygiene routine by athletes in the locker room. The DTPA-containing shower gel prevents biofilm-related infections such as MRSA or MRSE.

### EXAMPLE 17

A hard surface cleaning composition is prepared containing 2 mM DTPA. The cleaning solution is used to soak hard objects such as surgical instruments or food preparation tools. Biofilm formation by bacteria having zinc adhesion modules is inhibited.

### EXAMPLE 18

A hard surface cleaning composition is prepared containing 2 mM DTPA. The cleaning solution is applied to pipeline systems. Application of the cleaning solution prevents the formation of environmental or industrial biofilms on the surface of the pipes.

### EXAMPLE 19

A prosthetic hip replacement is prepared prior to surgery by bathing the device for 60 minutes in a sterile solution containing a 25 µM concentration of a protein construct comprising one zinc adhesion module. When the surgeon is ready to insert the prosthetic hip replacement, the device is removed from the sterile zinc adhesion module solution and implanted into the patient. Treatment with the zinc adhesion module solution inhibits biofilm formation on the prosthetic device.

### EXAMPLE 20

During the surgical installation of an implantable medical device, the vicinity of the surgical operation is rinsed with a sterile buffered saline solution containing a 2 µM concentration of a protein construct comprising six zinc adhesion modules. Upon the conclusion of the surgery, the sterile solution is aspirated from the surgical site. Rinsing with the zinc adhesion module solution inhibits biofilm formation on the implantable device by commensal or environmental bacteria that gain access to the implantation site during surgery.

## Claims

1. An *in vitro* method for inhibiting formation of a biofilm comprising bacteria, the method comprising contacting the bacteria with an effective amount of at least one zinc chelator, wherein the bacteria comprise at least one zinc adhesion module and wherein the bacteria are selected from the group consisting of *Staphylococcus epidermidis* and *Staphylococcus aureus* and combinations thereof, whereby formation of the biofilm is inhibited; **characterized in that** the at least one zinc chelator comprises DTPA.

2. The method of Claim 1 wherein the bacteria are selected from one or more of the group consisting of methicillin resistant *Staphylococcus aureus* (MRSA) and methicillin resistant *Staphylococcus epidermidis* (MRSE) and combinations thereof.

3. A method for inhibiting formation of a biofilm on a device, wherein the biofilm comprises bacteria comprising at least one zinc adhesion module and wherein the bacteria are selected from the group consisting of *Staphylococcus epidermidis* and *Staphylococcus aureus* and combinations thereof, the method comprising contacting the device with a solution comprising an effective amount of at least one zinc chelator, whereby formation of a biofilm on the device is inhibited; **characterized in that** the at least one zinc chelator comprises DTPA.

4. The method of Claim 3 wherein the device comprises an implantable medical device.

5. The method of Claim 4 wherein the implantable medical device is selected from the group consisting of pacemakers, heart valves, replacement joints, catheters, catheter access ports, dialysis tubing, gastric bands, shunts, screw plates, artificial spinal disc replacements, internal implantable defibrillators, cardiac resynchronization therapy devices, implantable cardiac monitors, mitral valve ring repair devices, left ventricular assist devices (LVADs), artificial hearts, implantable infusion pumps, implantable insulin pumps, stents, implantable neurostimulators, maxillofacial implants, and dental implants.

6. The method of any of Claims 3 to 5 wherein the contacting comprises bathing or coating the device.

7. The method of any of Claims 3 to 6 wherein the solution is a gel or polymer coating.

8. A topical pharmaceutical composition comprising a therapeutically effective amount of at least one zinc chelator and at least one pharmaceutically acceptable carrier, for use in a method of inhibiting formation of a biofilm in a mammal, wherein the biofilm comprises bacteria comprising at least one zinc adhesion module and wherein the bacteria are selected from the group consisting of *Staphylococcus epidermidis* and *Staphylococcus aureus* and combinations thereof; **characterized in that** the at least one zinc chelator comprises DTPA.

9. The pharmaceutical composition of Claim 8, further comprising a therapeutically effective amount of at least one antimicrobial agent, including an antibiotic.

10. The pharmaceutical composition of Claim 8 or Claim 9, wherein the composition is in the form of a surgical rinse, dental rinse, spray, solution, gel, cream, ointment, body wash, soap, surgical scrub, or impregnated bandage.

11. The method of any of Claims 1 to 7, wherein the concentration of each chelator is from 20 µM to 3 mM.

12. The pharmaceutical composition of any of Claims 8 to 10, wherein the concentration of each chelator is from 20 µM to 3 mM.

## Patentansprüche

1. In-vitro-Verfahren zum Hemmen der Bildung eines Biofilms, der Bakterien umfasst, wobei das Verfahren das In-Kontakt-Bringen der Bakterien mit einer wirksamen Menge von mindestens einem Zinkchelatbildner umfasst, wobei die Bakterien mindestens ein Zinkadhäsionsmolekül umfassen, und wobei die Bakterien aus der Gruppe ausgewählt sind, die aus Staphylococcus epidermidis und Staphylococcus aureus und Kombinationen davon besteht, wodurch die Bildung des Biofilms gehemmt wird, **dadurch gekennzeichnet, dass** der mindestens eine Zinkchelatbildner DTPA umfasst.

2. Verfahren gemäß Anspruch 1, wobei die Bakterien aus einem oder mehr aus der Gruppe ausgewählt sind, die aus methicillinresistentem Staphylococcus aureus (MRSA) und methicillinresistentem Staphylococcus epidermidis (MRSE) und Kombinationen davon besteht.

3. Verfahren zum Hemmen der Bildung eines Biofilms auf einem Produkt, wobei der Biofilm Bakterien umfasst, die mindestens ein Zinkadhäsionsmolekül umfassen, und wobei die Bakterien aus der Gruppe ausgewählt sind, die aus Staphylococcus epidermidis und Staphylococcus aureus und Kombinationen davon besteht, wobei das Verfahren das In-Kontakt-Bringen des Produkts mit einer Lösung umfasst, die eine wirksame Menge von mindestens einem Zinkchelatbildner umfasst, wodurch die Bildung eines Biofilms auf dem Produkt gehemmt wird; **dadurch gekennzeichnet, dass** der mindestens eine Zinkchelatbildner DTPA umfasst.

4. Verfahren gemäß Anspruch 3, wobei das Produkt ein implantierbares Medizinprodukt umfasst.

5. Verfahren gemäß Anspruch 4, wobei das implantierbare Medizinprodukt aus der Gruppe ausgewählt ist, die aus Folgenden besteht: Herzschrittmachern, Herzklappen, Gelenkersatz, Kathetern, Katheterzugangsports, Dialyseschläuchen, Magenbändern, Shunts, Schraubenplatten, künstlichem Bandscheibenersatz, internen implantierbaren Defibrillatoren, Produkten für die Herzresynchronisationstherapie, implantierbaren Herzmonitoren, Produkten für die Mitralklappenringreparatur, linksventrikulären Unterstützungssystemen (LVADs), künstlichen Herzen, implantierbaren Infusionspumpen, implantierbaren Insulinpumpen, Stents, implantierbaren Neurostimulatoren, Mund-Kiefer-Gesichts-Implantaten und Zahnimplantaten.

6. Verfahren gemäß einem der Ansprüche 3 bis 5, wobei das In-Kontakt-Bringen das Einlegen des Produkts in ein Bad oder das Beschichten des Produkts umfasst.

7. Verfahren gemäß einem der Ansprüche 3 bis 6, wobei die Lösung ein Gel oder eine Polymerbeschichtung ist.

8. Topische pharmazeutische Zusammensetzung, die eine therapeutisch wirksame Menge von mindestens einem Zinkchelatbildner und mindestens einem pharmazeutisch akzeptablen Träger umfasst, zur Verwendung in einem Verfahren des Hemmens der Bildung eines Biofilms bei einem Säuger, wobei der Biofilm Bakterien umfasst, die mindestens ein Zinkadhäsionsmolekül umfassen, und wobei die Bakterien aus der Gruppe ausgewählt sind, die aus Staphylococcus epidermidis und Staphylococcus aureus und Kombinationen davon besteht; **dadurch gekennzeichnet, dass** der mindestens eine Zinkchelatbildner DTPA umfasst.

9. Pharmazeutische Zusammensetzung gemäß Anspruch 8, die ferner eine therapeutisch wirksame Menge von mindestens einem antimikrobiellen Mittel, einschließlich eines Antibiotikums, umfasst.

10. Pharmazeutische Zusammensetzung gemäß Anspruch 8 oder 9, wobei die Zusammensetzung in Form einer chirurgischen Spülung, Zahnspülung, eines Sprays, einer Lösung, eines Gels, einer Creme, Salbe, Körperwaschflüssigkeit, Seife, chirurgischem Desinfektionsmittel oder eines imprägnierten Verbandstoffs ist.

11. Verfahren gemäß einem der Ansprüche 1 bis 7, wobei die Konzentration jedes Chelatbildners 20 µM bis 3 mM beträgt.

12. Pharmazeutische Zusammensetzung gemäß einem der Ansprüche 8 bis 10, wobei die Konzentration jedes Chelatbildners 20 µM bis 3 mM beträgt.

## Revendications

1. Procédé *in vitro* d'inhibition de la formation d'un biofilm comprenant des bactéries, le procédé comprenant la mise en contact des bactéries avec une quantité efficace d'au moins un chélateur de zinc, les bactéries comprenant au moins un module d'adhésion du zinc et les bactéries étant choisies dans le groupe constitué par *Staphylococcus epidermidis* et *Staphylococcus aureus* et leurs combinaisons, moyennant quoi la formation du biofilm est inhibée ; **caractérisé en ce qu'**au moins un chélateur de zinc comprend du DTPA.

2. Procédé selon la revendication 1, les bactéries étant choisies parmi une ou plusieurs du groupe constitué par *Staphylococcus aureus* résistant à la méticilline (MRSA) et *Staphylococcus epidermidis* résistant à la méticilline (MRSE) et leurs combinaisons.

3. Procédé *in vitro* d'inhibition de la formation d'un biofilm sur un dispositif, le biofilm comprenant des bactéries comprenant au moins un module d'adhésion du zinc et les bactéries étant choisies dans le groupe constitué par *Staphylococcus epidermidis* et *Staphylococcus aureus* et leurs combinaisons, le procédé comprenant la mise en contact du dispositif avec une solution comprenant une quantité efficace d'au moins un chélateur de zinc, moyennant quoi la formation d'un biofilm sur le dispositif est inhibée ; **caractérisé en ce qu'**au moins un chélateur de zinc comprend du DTPA.

4. Procédé selon la revendication 3, le dispositif comprenant un dispositif médical implantable.

5. Procédé selon la revendication 4, le dispositif médical implantable étant choisi dans le groupe constitué par les stimulateurs cardiaques, les valves cardiaques, les dispositifs d'arthroplastie, les cathéters, les orifices d'accès de cathéter, les tubulures pour dialyse, les anneaux gastriques, les shunts, les plaques à vis, les prothèses de disques vertébraux, les défibrillateurs implantables internes, les dispositifs de thérapie de resynchronisation cardiaque, les moniteurs cardiaques implantables, les dispositifs de réparation de l'anneau mitral, les dispositifs d'assistance ventriculaire gauche (DAVG), les coeurs artificiels, les pompes à perfusion implantables, les pompes à insuline implantables, les endoprothèses vasculaires, les neurostimulateurs implantables, les implants maxillo-faciaux et les implants dentaires

6. Procédé selon l'une quelconque des revendications 3 à 5, la mise en contact comprenant le mouillage ou le recouvrement du dispositif.

7. Procédé selon l'une quelconque des revendications 3 à 6, la solution étant un gel ou un revêtement polymère.

8. Composition pharmaceutique topique comprenant une quantité thérapeutiquement efficace d'au moins un chélateur de zinc et au moins un support pharmaceutiquement acceptable, pour une utilisation dans un procédé d'inhibition de la formation d'un biofilm chez un mammifère, le biofilm comprenant des bactéries comprenant au moins un module d'adhésion du zinc et les bactéries étant choisies dans le groupe constitué par *Staphylococcus epidermidis* et *Staphylococcus aureus* et leurs combinaisons ; **caractérisé en ce qu'**au moins un chélateur de zinc comprend du DTPA.

9. Composition pharmaceutique selon la revendication 8, comprenant en outre une quantité thérapeutiquement efficace d'au moins un agent antimicrobien, notamment un antibiotique.

10. Composition pharmaceutique selon la revendication 8 ou 9, la composition étant sous la forme d'un liquide de rinçage chirurgical, d'un liquide de rinçage dentaire, d'un spray, d'une solution, d'un gel, d'une crème, d'un onguent, d'un nettoyant corporel, d'un savon, d'un liquide de lavage chirurgical ou d'un pansement imprégné.

11. Procédé selon l'une quelconque des revendications 1 à 7, la concentration de chaque chélateur étant comprise entre 20 µM et 3 mM.

12. Composition pharmaceutique selon l'une quelconque des revendications 8 à 10, la concentration de chaque chélateur étant comprise entre 20 µM et 3 mM.


### SEQUENCE LISTING

<110> Herr, Andrew B.
   Conrady, Deborah G.
   Brescia, Cristin C.
   Ward, Stefanie L.
<120> Use of Zinc Chelators to Inhibit Biofilm Formation
<130> 10738-132
<150> 61/057267
   <151> 2008-05-30
<160> 2
<170> PatentIn version 3.5
<210> 1
   <211> 208
   <212> PRT
   <213> Staphylococcus epidermidis
<220>
   <221> MISC_FEATURE
   <222> (1)..(208)
   <223> Single complete zinc adhesion module including C-terminal capping sequence from the Accumulation-associated protein (Aap).
<400> 1
   Val Asp Gly Asp Ser Ile Thr Ser Thr Glu Glu Ile Pro Phe Asp Lys
   1 5 10 15
   Lys Arg Glu Phe Asp Pro Asn Leu Ala Pro Gly Thr Glu Lys Val Val
   20 25 30
   Gln Lys Gly Glu Pro Gly Thr Lys Thr Ile Thr Thr Pro Thr Thr Lys
   35 40 45
   Asn Pro Leu Thr Gly Glu Lys Val Gly Glu Gly Glu Pro Thr Glu Lys
   50 55 60
   Ile Thr Lys Gln Pro Val Asp Glu Ile Val His Tyr Gly Gly Glu Gln
   65 70 75 80
   Ile Pro Gln Gly His Lys Asp Glu Phe Asp Pro Asn Ala Pro Val Asp
   85 90 95
   Ser Lys Thr Glu Val Pro Gly Lys Pro Gly Val Lys Asn Pro Asp Thr
   100 105 110
   Gly Glu Val Val Thr Pro Pro Val Asp Asp Val Thr Lys Tyr Gly Pro
   115 120 125
   Val Asp Gly Asp Ser Ile Thr Ser Thr Glu Glu Ile Pro Phe Asp Lys
   130 135 140
   Lys Arg Glu Phe Asp Pro Asn Leu Ala Pro Gly Thr Glu Lys Val Val
   145 150 155 160
   Gln Lys Gly Glu Pro Gly Thr Lys Thr Ile Thr Thr Pro Thr Thr Lys
   165 170 175
   Asn Pro Leu Thr Gly Glu Lys Val Gly Glu Gly Lys Ser Thr Glu Lys
   180 185 190
   Val Thr Lys Gln Pro Val Asp Glu Ile Val Glu Tyr Gly Pro Thr Lys
   195 200 205
<210> 2
   <211> 208
   <212> PRT
   <213> Staphylococcus aureus
<220>
   <221> MISC_FEATURE
   <222> (1)..(208)
   <223> Single complete zinc adhesion module including C-terminal capping sequence from the SasG protein.
<400> 2
   Val Lys Gly Asp Ser Ile Val Glu Lys Glu Glu Ile Pro Phe Glu Lys
   1               5                   10                  15
   Glu Arg Lys Phe Asn Pro Asp Leu Ala Pro Gly Thr Glu Lys Val Thr
               20                  25                  30
   Arg Glu Gly Gln Lys Gly Glu Lys Thr Ile Thr Thr Pro Thr Leu Lys
           35                  40                  45
   Asn Pro Leu Thr Gly Glu Ile Ile Ser Lys Gly Glu Ser Lys Glu Glu
       50                  55                  60
   Ile Thr Lys Asp Pro Val Asn Glu Leu Thr Glu Phe Gly Gly Glu Lys
   65                  70                  75                  80
   Ile Pro Gln Gly His Lys Asp Ile Phe Asp Pro Asn Leu Pro Thr Asp
                   85                  90                  95
   Gln Thr Glu Lys Val Pro Gly Lys Pro Gly Ile Lys Asn Pro Asp Thr
               100                 105                 110
   Gly Lys Val Ile Glu Glu Pro Val Asp Asp Val Ile Lys His Gly Pro
           115                 120                 125
   Lys Thr Gly Thr Pro Glu Thr Lys Thr Val Glu Ile Pro Phe Glu Thr
       130                 135                 140
   Lys Arg Glu Phe Asn Pro Lys Leu Gln Pro Gly Glu Glu Arg Val Lys
   145                 150                 155                 160
   Gln Glu Gly Gln Pro Gly Ser Lys Thr Ile Thr Thr Pro Ile Thr Val
                   165                 170                 175
   Asn Pro Leu Thr Gly Glu Lys Val Gly Glu Gly Gln Pro Thr Glu Glu
               180                 185                 190
   Ile Thr Lys Gln Pro Val Asp Lys Ile Val Glu Phe Gly Gly Glu Lys
           195                 200                 205